(19) **Europäisches Patentamt / European Patent Office / Office européen des brevets**

(11) **EP 4 427 112 B1**

(12) **EUROPEAN PATENT SPECIFICATION**

(45) Date of publication and mention
of the grant of the patent:
**31.12.2025 Bulletin 2026/01**

(21) Application number: **22817528.7**

(22) Date of filing: **03.11.2022**

(51) International Patent Classification (IPC):
**G05D 23/19** *(2006.01)*  **G05D 23/22** *(2006.01)*
**C07C 5/333** *(2006.01)*  **C07C 11/09** *(2006.01)*

(52) Cooperative Patent Classification (CPC):
(C-Sets available)
**C07C 5/333**  (Cont.)

(86) International application number:
**PCT/EP2022/080669**

(87) International publication number:
**WO 2023/078994 (11.05.2023 Gazette 2023/19)**

(54) **PROGRAMMABLE LOGIC CONTROL OPTIMIZATION IN ISO-BUTANE DEHYDROGENATION UNITS FOR REACTOR SEQUENCE PRODUCTIVITY AND SAFETY**

OPTIMIERUNG DER PROGRAMMIERBAREN LOGIKSTEUERUNG IN ISO-BUTANDEHYDRIERUNGSEINHEITEN FÜR DIE REAKTORSEQUENZPRODUKTIVITÄT UND -SICHERHEIT

OPTIMISATION DE COMMANDE LOGIQUE PROGRAMMABLE DANS DES UNITÉS DE DÉSHYDROGÉNATION DE L'ISO-BUTANE POUR LA PRODUCTIVITÉ ET LA SÉCURITÉ DE SÉQUENCE DE RÉACTEUR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **03.11.2021 EP 21206125**

(43) Date of publication of application:
**11.09.2024 Bulletin 2024/37**

(73) Proprietor: **SABIC Global Technologies B.V.**
**4612 PX Bergen op Zoom (NL)**

(72) Inventors:
• **BENASKAR, Faysal**
**Bengaluru 562125 (IN)**
• **COMOTTI, Massimiliano**
**Bengaluru 562125 (IN)**
• **AL-MUTAIRI, Sami Muteib**
**Bengaluru 562125 (IN)**
• **AL-SHAFAI, Adel Saud**
**Bengaluru 562125 (IN)**
• **SHAHUL, Syed Hameed**
**Bengaluru 562125 (IN)**
• **RAMIREZ, Cesar Enrique**
**Bengaluru 562125 (IN)**
• **AL-KUDAISI, Ali**
**Bengaluru 562125 (IN)**
• **AL-AHMARI, Faisal**
**Bengaluru 562125 (IN)**

(74) Representative: **EP&C**
**P.O. Box 3241**
**2280 GE Rijswijk (NL)**

(56) References cited:
**WO-A1-2020/183359  US-A- 2 298 399**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 5/333, C07C 11/09**

**Description**

**CROSS REFERENCE TO RELATED APPLICATIONS**

None.

**FIELD OF INVENTION**

The present invention generally relates to systems and methods for hydrocarbon dehydrogenation. More specifically, the present invention relates to systems and methods for hydrocarbon dehydrogenation that include a control mechanism for switching one or more dehydrogenation reactors between a dehydrogenation mode and a regeneration mode based on temperatures in catalyst beds.

**BACKGROUND OF THE INVENTION**

Hydrocarbon dehydrogenation is a process used for producing a variety of alkene products. Examples of these alkene products include isobutylene used for producing methyl tert-butyl ether (MTBE) and propylene used for polypropylene production. Currently, most commercially available alkane dehydrogenation technologies, including Süd-Chemie CATOFIN® process, CATADIENE® process, UOP's Oleflex® process, Phillips' Star™ process, and the Snamprogetti-Yarsintez process, use fixed bed or fluidized bed reactors with various types of catalysts.

These hydrocarbon dehydrogenation units usually include multiple reactors containing catalyst beds therein. During the dehydrogenation process, each of the reactors are operated in cycles, each of which includes a dehydrogenation step, a catalyst regeneration step, a long evacuation/reduction step, and a steam purge-short evacuation step. Conventionally, the operation sequence for each of the reactors, including the timing and duration of each of the steps, is determined based on predicted catalyst activities, safety requirements, and mechanical properties of the reactors. However, the overall productivity of the hydrocarbon dehydrogenation units is generally not taken into consideration when the operation sequence is determined, resulting in loss of productivity of the hydrocarbon dehydrogenation units. Document US 2 298 399A1 discloses a device and method for controlling the temperature in a catalyst bed during reactivation of the catalyst bed. Document WO 2020/183359A1 discloses a method for controlling the switching between regeneration and dehydrogenation in multiple fixed bed dehydrogenation reactors using a programmable controller.

Overall, while systems and methods for dehydrogenating hydrocarbons exist, the need for improvements in this field persists in light of at least the aforementioned drawback of the conventional systems and methods.

**BRIEF SUMMARY OF THE INVENTION**

A solution to at least the above mentioned problem associated with the systems and methods for dehydrogenating hydrocarbons is discovered. The solution resides in a system and method for dehydrogenating hydrocarbons that includes a control mechanism at least partially based on productivity of the hydrocarbon dehydrogenation units in the system, thereby increasing the overall productivity and reducing the production costs for dehydrogenated hydrocarbon. Additionally, the disclosed methods and systems include measuring temperatures in the catalyst bed as the variable to represent the state of operation for the reactors to determine the operation sequence. This can be beneficial for avoiding any complicated procedure to obtain required measurements to determine how the reactors are operating, resulting in low capital expenditure and ease of implementation for the disclosed methods. Therefore, the systems and methods of the present invention provide a technical solution to the problem associated with the conventional systems and methods for dehydrogenating hydrocarbons.

Embodiments of the invention include a method of dehydrogenating a hydrocarbon. The method comprises initiating a dehydrogenation mode for a reactor to dehydrogenate the hydrocarbon. The method comprises detecting a temperature in a catalyst bed of the reactor during the dehydrogenation mode. The method comprises calculating mathematical time-derivatives of the detected temperature. The method comprises switching the reactor from the dehydrogenation mode to a regeneration mode to regenerate the catalyst of the catalyst bed, in response to an absolute value of the mathematical time-derivative of the detected temperature in the catalyst bed being less than a critical value.

Embodiments of the invention include a method of operating a fixed bed reactor for dehydrogenating a hydrocarbon. The method comprises initiating a dehydrogenation mode for the fixed bed reactor to dehydrogenate the hydrocarbon to produce a dehydrogenated hydrocarbon. The method comprises detecting a first temperature of a top portion of a catalyst bed and detecting a second temperature of a bottom portion of the catalyst bed of the fixed bed reactor. The method comprises calculating mathematical time-derivatives of the detected first temperature and mathematical time-derivatives of the detected second temperature. The method comprises switching the fixed bed reactor from the dehydrogenation mode to a regeneration mode to regenerate the catalyst bed, in response to (a) an absolute value of the mathematical time-derivative of the detected first temperature being less than a first critical value and/or (b) an absolute value of the mathematical time-derivative of the detected second temperature being less than a second critical value. The method comprises switching the fixed bed reactor from the regeneration mode to the dehydrogenation mode in response to (c) an absolute value of the mathematical time-derivative of the detected first temperature being less than a third critical value and/or (d) an absolute value of the mathematical time-derivative of the detected second temperature being less than a fourth critical value.

Embodiments of the invention include a method of operating a reaction unit comprising one or more fixed bed reactors for dehydrogenating a hydrocarbon. The method comprises providing a programmable logic controller (PLC). The PLC comprises (i) a memory, (ii) a database stored in the memory, the database comprising a first critical value, a second critical value, a third critical value and a fourth critical value, and (iii) one or more processors communicatively coupled to the memory, the one or more processors configured to switch each of the one or more fixed bed reactors between a dehydrogenation mode and a regeneration mode based on the first critical value, the second critical value, the third critical value, and the fourth critical value. The method comprises initiating, by the PLC, a dehydrogenation mode for one or more of the fixed bed reactors to dehydrogenate the hydrocarbon to produce a dehydrogenated hydrocarbon. The method comprises continuously detecting a first temperature of a top portion a catalyst bed of each of the fixed bed reactors. The method comprises continuously detecting a second temperature of a bottom portion of the catalyst bed of each of the fixed bed reactors. The method comprises continuously calculating, by the PLC, mathematical time-derivatives of the detected first temperature and the detected second temperature for each of the fixed bed reactors. The method comprises switching, via the PLC, one or more of the fixed bed reactors from the dehydrogenation mode to a regeneration mode to regenerate the catalyst bed of each of the fixed bed reactors, in response to (a) an absolute value of the mathematical time-derivative of the detected first temperature being less than a first critical value and/or (b) an absolute value of the mathematical time-derivative of the detected second temperature being less than a second critical value. The method comprises switching, via the PLC, one or more of the fixed bed reactors from the regeneration mode to the dehydrogenation mode, in response to (c) an absolute value of the mathematical time-derivative of the detected first temperature being less than a third critical value and/or (d) an absolute value of the mathematical time-derivative of the detected second temperature being less than a fourth critical value.

The following includes definitions of various terms and phrases used throughout this specification.

The terms "about" or "approximately" are defined as being close to as understood by one of ordinary skill in the art. In one non-limiting embodiment the terms are defined to be within 10%, preferably, within 5%, more preferably, within 1%, and most preferably, within 0.5%.

The terms "wt.%", "vol.%" or "mol.%" refer to a weight, volume, or molar percentage of a component, respectively, based on the total weight, the total volume, or the total moles of material that includes the component. In a non-limiting example, 10 moles of component in 100 moles of material is 10 mol.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification, include any measurable decrease or complete inhibition to achieve a desired result.

The term "effective," as that term is used in the specification and/or claims, means adequate to accomplish a desired, expected, or intended result.

The use of the words "a" or "an" when used in conjunction with the term "comprising," "including," "containing," or "having" in the claims or the specification may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The process of the present invention can "comprise," "consist essentially of," or "consist of" particular ingredients, components, compositions, etc., disclosed throughout the specification.

The term "primarily," as that term is used in the specification and/or claims, means greater than any of 50 wt.%, 50 mol.%, and 50 vol.%. For example, "primarily" may include 50.1 wt.% to 100 wt.% and all values and ranges there between, 50.1 mol.% to 100 mol.% and all values and ranges there between, or 50.1 vol.% to 100 vol.% and all values and ranges there between.

Other objects, features and advantages of the present invention will become apparent from the following figures, detailed description, and examples. It should be understood, however, that the figures, detailed description, and examples, while indicating specific embodiments of the invention, are given by way of illustration only and are not meant to be limiting. Additionally, it is contemplated that changes and modifications within the scope of the invention will become apparent to those skilled in the art from this detailed description. In further embodiments, features from specific embodiments may be combined with features from other embodiments. For example, features from one embodiment may be combined with features from any of the other embodiments. In further embodiments, additional features may be added to the specific embodiments described herein.

## BRIEF DESCRIPTION OF THE DRAWINGS

For a more complete understanding, reference is now made to the following descriptions taken in conjunction with the accompanying drawings, in which:

FIG. 1 shows a schematic diagram for a system of dehydrogenating a hydrocarbon, according to embodiments of the invention;

FIG. 2 shows a schematic flowchart for a method of dehydrogenating a hydrocarbon, according to embodiments of the invention;

FIG. 3A shows temperature profiles for a top portion and a bottom portion of a catalyst bed in a dehydrogenation reactor during start of run for a 24-months operation;

FIG. 3B shows temperature profiles for a top portion and a bottom portion of a catalyst bed in a dehydrogenation reactor during end of run for a 24-months operation;

FIG. 4A shows temperature profiles for start of run and end of run of a top portion of a catalyst bed in a dehydrogenation reactor during start of run for a single dehydrogenation-regeneration cycle;

FIG. 4B shows temperature profiles for start of run and end of run of a bottom portion of a catalyst bed in a dehydrogenation reactor during start of run for a single dehydrogenation-regeneration cycle;

FIG. 5A shows linear regression results for temperature profiles of a top portion and a bottom portion of a catalyst bed in a dehydrogenation reactor during start of run; and

FIG. 5B shows linear regression results for temperature profiles of a top portion and a bottom portion of a catalyst bed in a dehydrogenation reactor during end of run.

## DETAILED DESCRIPTION OF THE INVENTION

Currently, the operation sequence (*e.g.,* for timing and duration for dehydrogenation and regeneration steps) for reactors in a hydrocarbon dehydrogenation system is determined based on catalyst activity and safety specifications without considering overall productivity of the reactors, resulting in low productivity and increased production cost for dehydrogenated hydrocarbons. The present invention provides a solution to at least this problem. The solution is premised on a method of dehydrogenating hydrocarbons where the reactors are sequenced based on productivity of the reactors such that the productivity of dehydrogenated hydrocarbon is optimized. Furthermore, the disclosed method uses temperatures in the catalyst beds of the reactors as an indication of productivity, thereby simplifying the steps to implement the methods in the dehydrogenation unit. These and other non-limiting aspects of the present invention are discussed in further detail in the following sections.

### A. System for dehydrogenating hydrocarbons

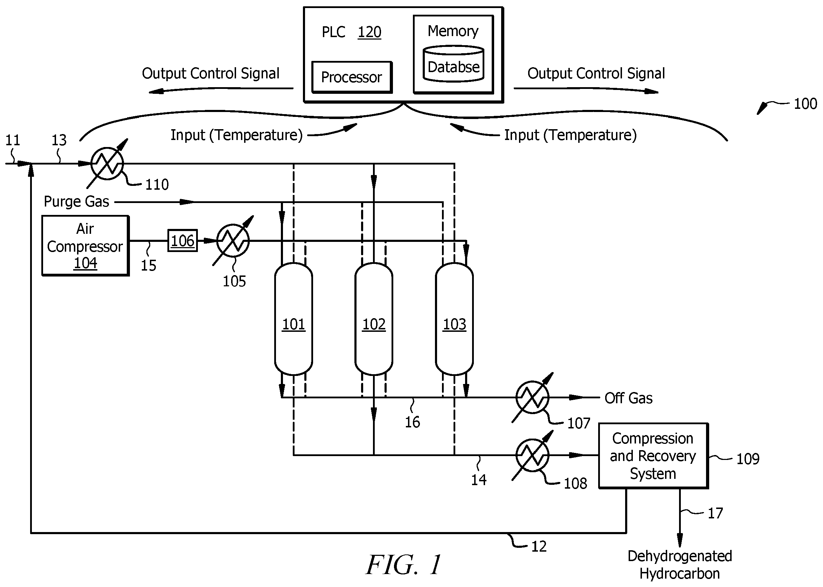

In embodiments of the invention, the system for dehydrogenating hydrocarbons includes one or more reactors and a control unit configured to control the sequence of the one or more reactors. With reference to FIG. 1, a schematic diagram is shown for system 100, which is used for dehydrogenating hydrocarbons.

According to embodiments of the invention, system 100 includes a reaction unit comprising one or more dehydrogenation reactors (e.g., reactor 101, reactor 102, and reactor 103) configured to dehydrogenate a hydrocarbon. Non-limiting examples of the hydrocarbon can include isobutane, n-butane, propane, ethane, methane, and combinations thereof. In embodiments of the invention, the dehydrogenation reactors are fixed bed reactors and each of the dehydrogenation reactors comprises a catalyst bed. In embodiments of the invention, the reaction unit is configured to dehydrogenate isobutane and the reaction unit comprises 3-5 fixed bed reactors operated in parallel. In embodiments of the invention, the reaction unit comprises 6-12 fixed bed reactors operated in parallel. In embodiments of the invention, the catalyst comprises oxides or carbides of Al, Si, Ti, Zr, Zn, Ce, Sn, Mg, Ca, La, Cr, Cs, Ba, and combinations thereof. The catalyst may include Cr/Al (chromium oxide over alumina), Sn-Pt/Al (tin-platinum over alumina), or combinations thereof.

Each of the reactors is configured to operate in a dehydrogenation mode, a regeneration mode, and a purging mode, according to embodiments of the invention.

In the dehydrogenation mode, the inlet of each of the reactors, in embodiments of invention, is in fluid communication with a heater 110 (which is configured to heat a hydrocarbon feed stream 13 to a reaction temperature), and the outlet of the reactors, in embodiments of the invention, is in fluid communication with a heat exchanger 108 (which is configured to cool a reaction effluent stream 14 coming from the reactors). In embodiments of the invention, system 100 comprises a compression and recovery system 109 configured to compress and separate reaction effluent stream 14 to produce a recycle stream 12 comprising unreacted hydrocarbon, and a product stream 17 comprising the dehydrogenated hydrocarbon. Recycle stream 12 and a fresh feed stream 11 comprising the hydrocarbon can be combined to form hydrocarbon feed stream 13.

System 100 may further include a regeneration air system comprising an air compressor 104 configured to blow an air stream 15 into the reactor in the regeneration mode. According to embodiments of the invention, a regeneration air heater 105 is configured to heat air stream 15 from air compressor 104. System 100 can comprise a fuel injector 106 configured to inject fuel gas into the reactor in the regeneration mode. System 100 can further comprise a heat exchanger 107 configured to cool down a regeneration effluent stream 16 from the reactor in the regeneration mode or in the purging mode.

According to embodiments of the invention, system 100 comprises a control system configured to control operating parameters of the reactors. The control system can include a plurality of temperature sensors configured to obtain temperature readings at one or more locations of the catalyst bed of each reactor, and a programmable logic controller (PLC) 120 configured to determine the sequence of the reactors, including timing and duration of the regeneration mode, the dehydrogenation mode, and/or the purging mode. In embodiments of the invention, PLC 120 is configured to determine the sequence of the reactors based on the temperature readings. In embodiments of the invention, the temperature sensors are configured to detect temperature on a top portion and a bottom portion of a catalyst bed of a reactor. The top portion of the catalyst bed is the upper half of the catalyst bed of each reactor. In embodiments of the invention, the top portion of the catalyst bed is a portion from top of the catalyst bed to 15 to 22% depth of the catalyst bed. The bottom portion of the catalyst bed is the lower half of the catalyst bed of each reactor. In embodiments of the invention, the bottom portion is a bottom 10% to 20% depth of the catalyst bed. In embodiments of the invention, the depth of the catalyst bed for each reactor is about 190 cm, and the top portion of the catalyst bed is the region from the top of the catalyst bed to 30 cm in the catalyst bed below the top of the catalyst bed, and the bottom portion of the catalyst bed includes 160 cm from the top of the catalyst bed to the bottom of the catalyst bed. In embodiments of the invention, the PLC 120 can include a proportional-integral-derivative (PID) controller.

In embodiments of the invention, PLC 120 can include a memory and a database stored in the memory. In embodiments of the invention, the database comprises a first critical value, a second critical value, a third critical value, and fourth critical value. In embodiments of the invention, PLC 120 comprises one or more processors communicatively coupled to the memory, the one or more processors configured to switch each of the fixed bed reactors between the dehydrogenation mode and the regeneration mode based on the first critical value, the second critical value, the third critical value, and fourth critical value. In embodiments of the invention, PLC 120 comprises a non-transitory storage medium having instructions for switching each of the fixed bed reactors between the dehydrogenation mode and the regeneration mode based on one or more of the first critical value, the second critical value, the third critical value, and fourth critical value.

According to embodiments of the invention, PLC 120 is configured to control each of the one or more fixed bed reactors independently. According to embodiments of the invention, the control system is configured to, via the temperature sensors, continuously, intermittently, or periodically detect a first temperature of a top portion of a catalyst bed of each of the reactors and continuously, intermittently, or periodically detect a second temperature of a bottom portion of the catalyst bed of each of the reactors during the dehydrogenation mode. The control system may be configured to detect the first temperature and/or the second temperature continuously or intermittently with an interval of 1 second to 10 minutes and all ranges and values there between including a ranges of 1 second to 10 seconds, 10 seconds to 20 seconds, 20 seconds to 30 seconds, 30 seconds to 40 seconds, 40 seconds to 50 seconds, 50 seconds to 1 minute, 1 minute, to 2 minute, 2 minutes to 3 minutes, 3 minutes to 4 minutes, 4 minutes to 5 minutes, 5 minutes to 6 minutes, 6 minutes to 7 minutes, 7 minutes to 8 minutes, 8 minutes to 9 minutes, an 9 minutes to 10 minutes. In embodiments of the invention, PLC 120 is configured to switch one or more of the fixed bed reactors from the dehydrogenation mode to a regeneration mode to regenerate the catalyst bed of each of the fixed bed reactors, in response to (a) an absolute value of the mathematical time-derivative of the detected first temperature being less than the first critical value and/or (b) an absolute value of the mathematical time-derivative of the detected second temperature being less than the second critical value. In embodiments of the invention, PLC 120 is configured to switch one or more of the fixed bed reactors from the regeneration mode to the dehydrogenation mode, in response to (c) an absolute value of the mathematical time-derivative of the detected first temperature being less than the third critical value and/or (d) an absolute value of the mathematical time-derivative of the detected second temperature being less than the fourth critical value.

## B. Method of dehydrogenating hydrocarbons

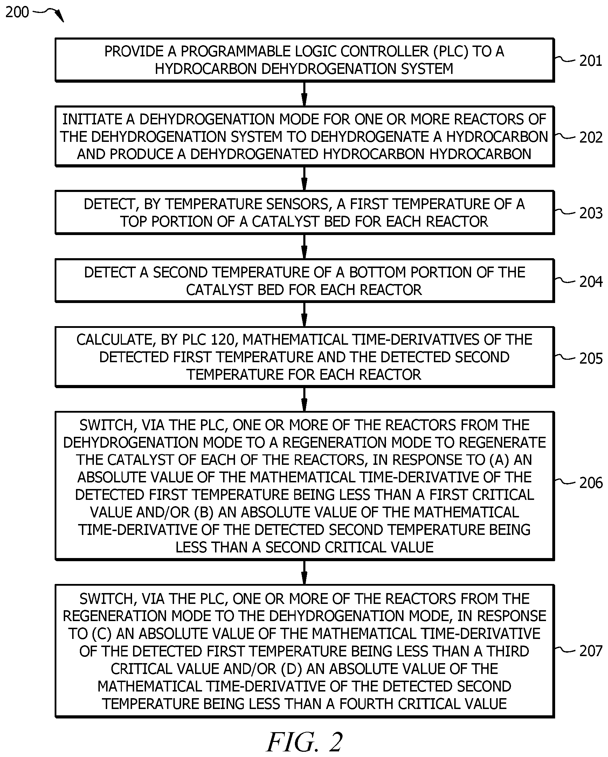

Methods of dehydrogenating a hydrocarbon have been discovered. As shown in FIG. 2, embodiments of the invention include method 200 for dehydrogenating hydrocarbons with improved productivity of dehydrogenated hydrocarbons compared to conventional methods. Method 200 may be implemented by system 100, as shown in FIG. 1 and described above.

According to embodiments of the invention, as shown in block 201, method 200 includes providing the programmable logic controller (PLC) 120 to a hydrocarbon dehydrogenation system. According to embodiments of the invention, as shown in block 202, method 200 includes initiating a dehydrogenation mode for one or more of the reactors of the dehydrogenation system to dehydrogenate a hydrocarbon and produce a dehydrogenated hydrocarbon. Non-limiting examples of the hydrocarbon can include isobutane, n-butane, propane, ethane, methane, and combinations thereof. In embodiments of the invention, initiating at block 202 includes flowing a feed stream comprising the hydrocarbon into the reactor at a reaction temperature, and contacting the hydrocarbon with the catalyst under reaction conditions sufficient to produce a product an effluent stream comprising the dehydrogenated hydrocarbon. In embodiments of the invention, the reaction conditions include a reaction temperature of 530 to 690 °C and all ranges and values there between including a reaction temperature of 530 to 540 °C, 540 to 550 °C, 550 to 560 °C, 560 to 570 °C, 570 to 580 °C, 580 to 590 °C, 590 to 600 °C, 600 to 610 °C, 610 to 620 °C, 620 to 630 °C, 630 to 640 °C, 640 to 650 °C, 650 to 660 °C, 660 to 670 °C, 670 to 680 °C, and 680 to 690 °C. The reaction conditions can include a reaction pressure of 0.2 to 1.8 bar and all ranges and values there between including ranges of 0.2 to 0.4 bar, 0.4 to 0.6 bar, 0.6 to 0.8 bar, 0.8 to 1.0 bar, 1.0 to 1.2 bar, 1.2 to 1.4 bar, 1.4 to 1.6 bar, and 1.6 to 1.8 bar. The reaction conditions can further include a weight hourly space velocity of 0.5 to 3.0 $hr^{-1}$, and all ranges and values there between including ranges of 0.5 to 1.0 $hr^{-1}$, 1.0 to 1.5 $hr^{-1}$, 1.5 to 2.0 $hr^{-1}$, 2.0 to 2.5 $hr^{-1}$, and 2.5 to 3.0 $hr^{-1}$. In embodiments of the invention, the initiating at block 202 is conducted via PLC 120.

According to embodiments of the invention, as shown in block 203, method 200 includes detecting, by the temperature sensors, a first temperature ($T_1$) of a top portion of a catalyst bed for each of the reactors. The detecting at block 203 can be conducted continuously. According to embodiments of the invention, as shown in block 204, method 200 includes detecting a second temperature ($T_2$) of a bottom portion of the catalyst bed for each of the reactors. In embodiments of the invention, the detecting at block 204 can be conducted continuously. Detecting at blocks 203 and 204 can be conducted simultaneously. As an alternative to being conducted continuously, the detecting at block 203 and/or block 204 can be conducted intermittently with an interval of 1 second to 10 minutes and all ranges and values there between including a ranges of 1 second to 10 seconds, 10 seconds to 20 seconds, 20 seconds to 30 seconds, 30 seconds to 40 seconds, 40 seconds to 50 seconds, 50 seconds to 1 minute, 1 minute, to 2 minute, 2 minutes to 3 minutes, 3 minutes to 4 minutes, 4 minutes to 5 minutes, 5 minutes to 6 minutes, 6 minutes to 7 minutes, 7 minutes to 8 minutes, 8 minutes to 9 minutes, an 9 minutes to 10 minutes.

According to embodiments of the invention, as shown in block 205, method 200 includes calculating, by PLC 120, mathematical time-derivatives of the detected first temperature ($dT_1/dt$) and the detected second temperature ($dT_2/dt$) for each of the reactors. In embodiments of the invention, calculating at block 205 is conducted continuously. In embodiments of the invention, in each cycle of the reactor (*i.e.,* regeneration mode and dehydrogenation mode), the correlation between the first temperature ($T_1$) and time can include two or more linear sections, preferably 6 linear sections. In embodiments of the invention, in each cycle of the reactor (*i.e.,* regeneration mode and dehydrogenation mode), the correlation between the second temperature ($T_2$) and time can include two or more linear sections, preferably 4 linear sections.

According to embodiments of the invention, as shown in block 206, method 200 includes switching, via PLC 120, one or more of the reactors from the dehydrogenation mode to a regeneration mode to regenerate the catalyst of each of the reactors, in response to (a) an absolute value of the mathematical time-derivative of the detected first temperature being less than a first critical value and/or (b) an absolute value of the mathematical time-derivative of the detected second temperature being less than a second critical value.

According to embodiments of the invention, as shown in block 207, method 200 includes switching, via PLC 120, one or more of the fixed bed reactors from the regeneration mode to the dehydrogenation mode, in response to (c) an absolute value of the mathematical time-derivative of the detected first temperature being less than a third critical value and/or (d) an absolute value of the mathematical time-derivative of the detected second temperature being less than a fourth critical value. In embodiments of the invention, the first critical value can be in a range of 8 to 14 °C/min. The second critical value can be in a range of 5 to 8 °C/min. The third critical value can be in a range of 2 to 5 °C/min. The fourth critical value can be in a range of 0.1 to 2 °C/min.

In embodiments of the invention, the first critical value, the second critical value, the third critical value, and the fourth critical value are each obtained from experimentation and/or simulation of the hydrocarbon dehydrogenation process. According to embodiments of the invention, the first critical value, the second critical value, the third critical value, and the fourth critical value can vary between Start of Run and End of Run for each reactor. In embodiments of the invention, blocks 202-207 are executed via instructions stored in a non-transitory storage medium in PLC 120.

Although embodiments of the present invention have been described with reference to blocks of FIG. 2 should be appreciated that operation of the present invention is not limited to the particular blocks and/or the particular order of the blocks illustrated in FIG. 2. Accordingly, embodiments of the invention may provide functionality as described herein using various blocks in a sequence different than that of FIG. 2.

The systems and processes described herein can also include various equipment that is not shown and is known to one of skill in the art of chemical processing. For example, some controllers, piping, computers, valves, pumps, heaters, thermocouples, pressure indicators, mixers, heat exchangers, and the like may not be shown.

As part of the disclosure of the present invention, specific examples are included below. The examples are for illustrative purposes only and are not intended to limit the invention. Those of ordinary skill in the art will readily recognize parameters that can be changed or modified to yield essentially the same results.

## EXAMPLE 1

### (Temperature profile for a catalyst bed of a dehydrogenation reactor during a 24-month run)

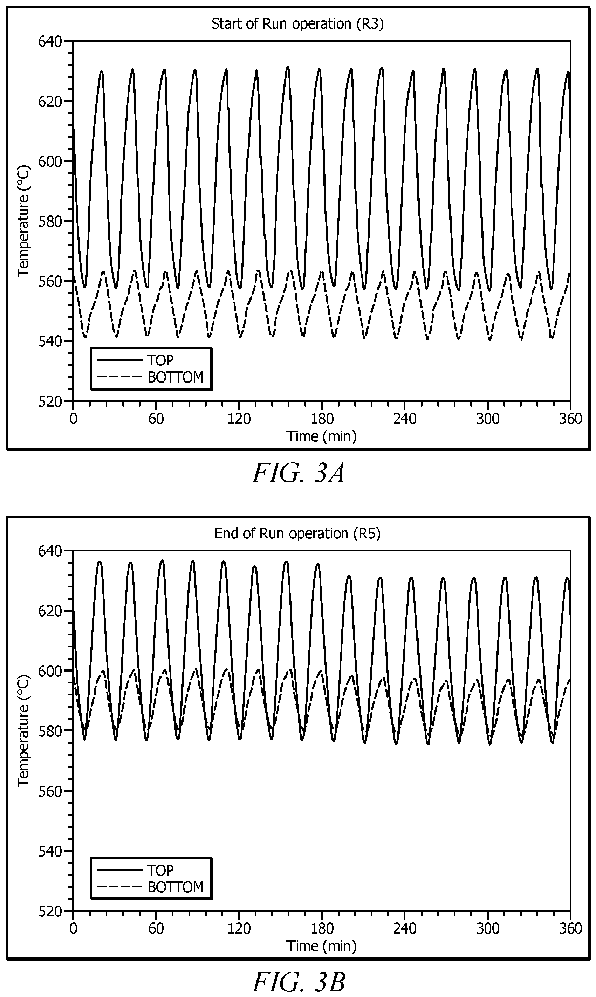

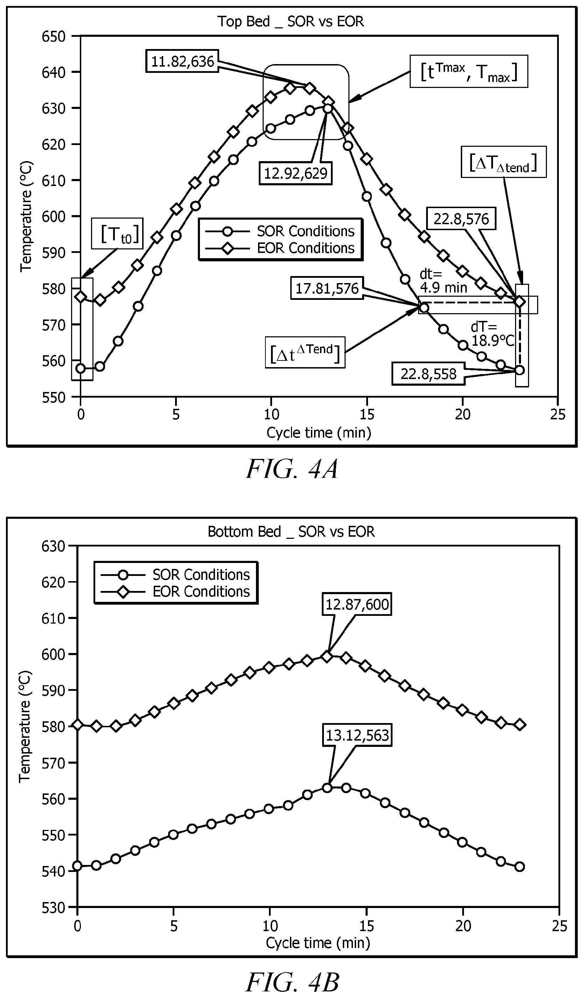

A temperature profile was plotted for a catalyst bed in a dehydrogenation reactor used for dehydrogenating isobutane during a 24-month run. Temperatures for both the top portion and the bottom portion of the catalyst bed were plotted against time. FIG. 3A shows the temperature profile at the Start of Run (SOR). FIG. 3B shows the temperature profile at the End of Run (EOR). FIG. 4A shows the temperature profile for a single cycle of the dehydrogenation run during the 24-month run at the top portion of the catalyst bed for Start of Run and End of Run. FIG. 4B shows the temperature profile for the corresponding bottom portion of the catalyst bed during the same cycle of the dehydrogenation run.

The operation conditions are defined as:

[SOR] means start of run conditions, typically 2nd month to 5th month of operation for the run;
[EOR] means end of run conditions, typically after 20th month of operation for the run.

Catalyst bed temperature locations are defined as:

[Top] means a location within a penetration of 30 cm in the catalyst section from the top of the catalyst bed; and
[Bot] means a location with a penetration of 160 cm in the catalyst bed.

Characteristics of these temperature profiles are defined as:

[$T_{t0}$] means the initial temperature at the start of regeneration.

Equation 1:

$$[T_{t0}^{SOR}] = 558\,°C; \quad [T_{t0}^{EOR}] = 577\,°C; \qquad \text{equation 1.1}$$

$$[\Delta T_{t0}^{EOR}] - [\Delta T_{t0}^{SOR}] = 19°C; \qquad \text{equation 1.2}$$

[$t^{Tmax}, T_{max}$] means time point at which maximum temperature is reached and the value of the maximum temperature.

Equation 2:

$$[t^{Tmax}, T_{max}]^{SOR} = [12.92, 629]; \qquad [t^{Tmax}, T_{max}]^{EOR} = [11.82, 636] \qquad \text{equation 2.1}$$

$$[t^{Tmax}]^{EOR} - [t^{Tmax}]^{SOR} = 12.92 - 11.82 = 1.10\ min \qquad \text{equation 2.2}$$

$$[T_{max}]^{EOR} - [T_{max}]^{SOR} = 636 - 629 = 7°C; \qquad \text{equation 2.3}$$

[$\Delta t^{\Delta Tend}$] means time difference which minimum temperature is reached at the end of the dehydrogenation mode.
Equation 3:

$$[\Delta t^{\Delta Tend}] = [t_{T=576}]^{EOR} - [t_{T=576}]^{SOR} = 22.80 - 17.81 = 4.99\ min \qquad \text{equation 3.1}$$

[$\Delta T_{\Delta tend}$] means temperature difference at the end of the dehydrogenation mode and the start of the regeneration mode.

Equation 4:

$$[\Delta T_{\Delta t_{end}}] = T_{t=22.80}]^{EOR} - [T_{t=22.80}]^{SOR} = 576 - 558 = 19°C.$$

## EXAMPLE 2

**(Analysis of temperature profile in catalyst bed of dehydrogenation reactor)**

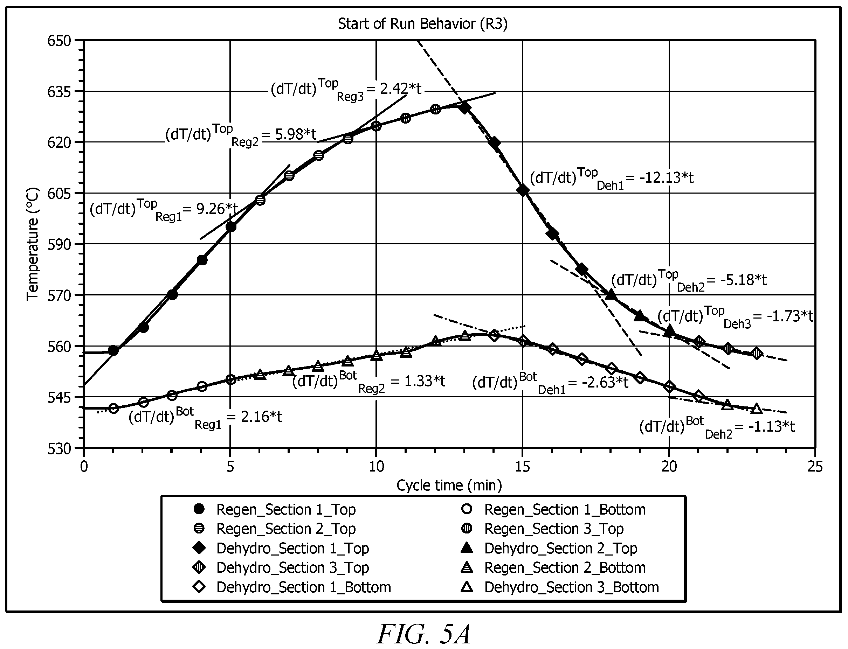

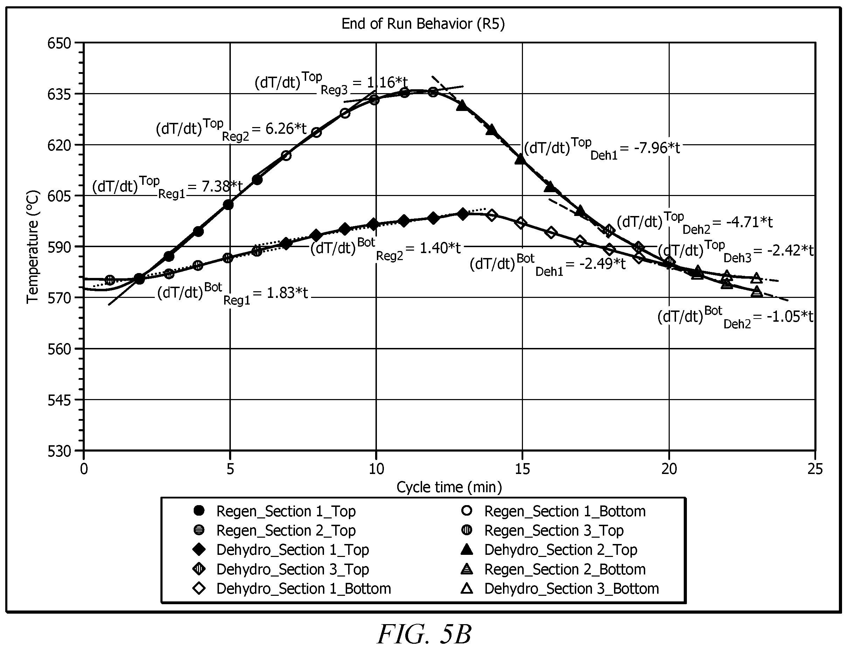

In the graphical representation in FIGs. 5A and 5B, the temperature profiles in the catalyst bed are compared between SOR (3 months) and EOR (22 months).

Mathematical approach of temperature profiles

As explained in the previous section, the temperature profiles demonstrate a significant variation in terms of the operational time and catalyst bed location.

FIG. 5A demonstrates the Start of Run temperature behavior in the top and bottom portions of a catalyst bed. As shown, the breakdown of the Exothermic (temperature rise) and the Endothermic (temperature drop) in the top portion of the bed can be done with three linearization sections, each section characterizing three slopes, respectively, for each of the regeneration and dehydrogenation modes. The temperature behavior of the bottom portion of the bed only requires two linearization sections for each of the regeneration and dehydrogenation modes due to the lower temperature response in the bottom portion of the bed. The mathematical time-derivative of temperature requires practically ten linearization sections to describe the bottom and top bed sections during the dehydrogenation and regeneration steps. The corresponding slope values of each of the linearization sections can be analyzed to define the effective temperature rise or drop in each linearization section. Similarly, for the End of Run operation, the temperature behavior in the top and bottom portions of the catalyst bed is shown in FIG. 5B. These sections of the temperature profiles shown in FIGS. 5A and 5B can be defined as:

$$\left\{\frac{dT}{dt}\right\}_{Reg\,n}^{Top} = \left\{\frac{\Delta T}{\Delta t}\right\}_{Reg\,n}^{Top} \quad \text{the linearization section slope of the regeneration temperature of the top}$$

portion of the catalyst bed, where n = 1, 2, and 3          equation 5.1;

$$\left\{\frac{dT}{dt}\right\}_{Deh\,n}^{Top} = \left\{\frac{\Delta T}{\Delta t}\right\}_{Deh\,n}^{Top} : \text{the linearization section slope of the dehydrogenation temperature of the}$$

top portion of the catalyst bed, where n = 1, 2, and 3          equation 5.2;

$$\left\{\frac{dT}{dt}\right\}_{Reg\,n}^{Bot} = \left\{\frac{\Delta T}{\Delta t}\right\}_{Reg\,n}^{Bot} : \text{the linearization section slope of the regeneration temperature of the}$$

bottom portion of the catalyst bed, where n = 1 and 2          equation 5.3;

$$\left\{\frac{dT}{dt}\right\}_{Deh\,n}^{Bot} = \left\{\frac{\Delta T}{\Delta t}\right\}_{Deh\,n}^{Bot} : \text{the linearization section slope of the dehydrogenation temperature of the}$$

bottom portion of the catalyst bed, where n = 1 and 2          equation 5.4.

The results of the temperature slope *(i.e., absolute values for the time derivatives of the temperatures)* for the Start of Run (shown in FIG. 5A) are summarized in Table 1.

Table 1

| Catalyst Top Bed | | | | | | Catalyst Bottom Bed | | | | | |
|---|---|---|---|---|---|---|---|---|---|---|---|
| $\left(\dfrac{\Delta T}{\Delta t}\right)^{Top}_{Reg\,n}$ | | | $\left(\dfrac{\Delta T}{\Delta t}\right)^{Top}_{Deh\,n}$ | | | $\left(\dfrac{\Delta T}{\Delta t}\right)^{Bot}_{Reg\,n}$ | | | $\left(\dfrac{\Delta T}{\Delta t}\right)^{Bot}_{Deh\,}$ | | |
| n = | Slope | % | n = | Slope | % | n = | Slope | % | n = | Slope | % |
| 1 | 9.26 | 100 | 1 | 12.13 | 100 | 1 | 2.16 | 100 | 1 | 2.63 | 100 |
| 2 | 5.98 | | 2 | 5.18 | | 2 | 1.33 | | 2 | 1.13 | |
| 3 | 2.42 | | 3 | 1.73 | | | | | | | |

According to Table 1 and FIGS. 5A and 5B, when the slope (absolute value of the mathematical time derivative of the temperature ) of the top portion of the catalyst bed during the regeneration mode was 2.42, the regeneration process was no longer efficient. When the slope (mathematical time derivative of the temperature) of the top portion of the catalyst bed during the dehydrogenation mode was 1.73, the dehydrogenation process was no longer efficient. Similarly, for the temperature of the bottom portion of the catalyst bed, when the slope was 1.33, the regeneration process was no longer efficient. When the slope for the bottom portion of the catalyst bed was 1.13, the dehydrogenation process was no longer efficient. The results show that the temperature time derivative based approach for switching between dehydrogenation and regeneration of the catalyst resulted in a gain of up to 5 minutes each cycle for the dehydrogenation. According to embodiments of the invention, the linearization can be conducted via linear regression. In an embodiment of the invention, the slope of the entire linearization section can be determined by linear regression. The time derivative of bed temperature (top portion and/or bottom portion) at each point may then be compared against the slope of the linear regression line. Substantial deviation from the slope of the linear regression line at any of the points may indicate that the regeneration process and/or the dehydrogenation process is no longer efficient at that point. As used herein, a 'substantial deviation' from the slope of the linear regression line is a slope having an absolute value greater than 5%, or greater than 7.5%, or greater than 10%, or greater than 12.5%, or greater than 15%, or greater than 20%, or greater than 25%, or greater than 30%, or greater than 35%, or greater than 40%, or greater than 45%, or greater than 50% of the slope of the linear regression line.

In the context of the present invention, at least the following 15 embodiments are described. Embodiment 1 is a method of dehydrogenating a hydrocarbon. The method includes initiating a dehydrogenation mode for a reactor to dehydrogenate the hydrocarbon. The method further includes detecting a temperature in a catalyst bed of the reactor during the dehydrogenation mode. The method still further includes calculating mathematical time-derivatives of the detected temperature, and switching the reactor from the dehydrogenation mode to a regeneration mode to regenerate the catalyst of the catalyst bed, in response to an absolute value of the mathematical time-derivative of the detected temperature in the catalyst bed being less than a critical value. Embodiment 2 is the method of embodiment 1, wherein the reactor is a fixed bed reactor. Embodiment 3 is the method of any of embodiments 1 and 2, wherein the detecting includes detecting a first temperature of a top portion of a catalyst bed and detecting a second temperature of a bottom portion of the catalyst bed of the reactor during the dehydrogenation mode. Embodiment 4 is the method of any of embodiments 1 to 3, wherein the top portion includes a portion from a top of the catalyst bed to 15 % to 22% depth of the catalyst bed, and the bottom portion includes a bottom 10% to 20% depth of the catalyst bed. Embodiment 5 is the method of any of embodiments 1 to 4, wherein the depth of the catalyst bed is about 190 cm, the top portion of the catalyst bed is a region from top of the catalyst bed to 30 cm in the catalyst bed below the top of the catalyst bed, and the bottom portion of the catalyst bed includes a region from 160 cm from top of the catalyst bed to the bottom of the catalyst bed. Embodiment 6 is the method of any of embodiments 1 to 5, wherein the calculating includes calculating mathematical time-derivatives of the detected first temperature and mathematical time-derivatives of the detected second temperature. Embodiment 7 is the method of any of embodiments 1 to 6, wherein the switching includes switching the reactor from the dehydrogenation mode to a regeneration mode to regenerate the catalyst bed, in response to (a) an absolute value of the mathematical time-derivative of the detected first temperature being less than a first critical value and/or (b) an absolute value of the mathematical time-derivative of the detected second temperature being less than a second critical value. Embodiment 8 is the method of any of embodiments 1 to 7, further including switching the reactor from the regeneration mode to the dehydrogenation mode in response to (c) an absolute value of the mathematical time-derivative of the detected first temperature being less than a third critical value and/or (d) an absolute value of the mathematical time-derivative of the detected second temperature being less than a fourth critical value. Embodiment 9 is the method of any of embodiments 1 to 8, wherein each of the initiating, detecting, calculating, and switching steps is conducted continuously. Embodiment 10 is the method of any of embodiments 1 to 9, wherein the hydrocarbon includes isobutane, n-butane, propane, ethane, methane, or combinations thereof.

Embodiment 11 is a method of operating a reaction unit containing one or more fixed bed reactors for dehydrogenating a hydrocarbon. The method includes providing a programmable logic controller (PLC) with: (i) a memory; (ii) a database stored in the memory, the database having a first critical value, a second critical value, a third critical value and a fourth critical value; and (iii) one or more processors communicatively coupled to the memory, the one or more processors configured to switch each of the one or more fixed bed reactors between a dehydrogenation mode and a regeneration mode based on the first critical value, the second critical value, the third critical value, and the fourth critical value. The method further includes initiating, by the PLC, the dehydrogenation mode for one or more of the fixed bed reactors to dehydrogenate the hydrocarbon to produce a dehydrogenated hydrocarbon. The method still further includes continuously detecting a first temperature of a top portion a catalyst bed of each of one or more of the fixed bed reactors. The method additionally includes continuously detecting a second temperature of a bottom portion of the catalyst bed of each of one or more of the fixed bed reactors during the dehydrogenation mode. The method also includes continuously calculating, by the PLC, mathematical time-derivatives of the detected first temperature and the detected second temperature for each of the fixed bed reactors. The method yet further includes switching, via the PLC, one or more of the fixed bed reactors from the dehydrogenation mode to the regeneration mode to regenerate the catalyst bed of each of the fixed bed reactors, in response to (a) an absolute value of the mathematical time-derivative of the detected first temperature being less than the first critical value and/or (b) an absolute value of the mathematical time-derivative of the detected second temperature being less than the second critical value. The method still further includes switching, via the PLC, one or more of the fixed bed reactors from the regeneration mode to the dehydrogenation mode, in response to (c) an absolute value of the mathematical time-derivative of the detected first temperature being less than the third critical value and/or (d) an absolute value of the mathematical time-derivative of the detected second temperature being less than the fourth critical value. Embodiment 12 is the method of embodiment 11, wherein the reaction unit is configured to dehydrogenate isobutane, wherein the reaction unit comprises 3-5 fixed bed reactors in parallel. Embodiment 13 is the method of any of embodiments 11 and 12, wherein the reaction unit is configured to dehydrogenate $C_3$ hydrocarbons, wherein the reaction unit comprises 6-12 fixed bed reactors in parallel. Embodiment 14 is the method of any of embodiments 11 to 13, wherein the PLC is configured to control each of the one or more fixed bed reactors independently. Embodiment 15 is the method of any of embodiments 11 to 14, wherein the catalyst is selected from the group consisting of oxides or carbides of Al, Si, Ti, Zr, Zn, Ce, Sn, Mg, Ca, La, Cr, Cs, Ba, and combinations thereof.

Although embodiments of the present application and their advantages have been described in detail, it should be understood that various changes, substitutions and alterations can be made herein without departing from the scope of the embodiments as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the above disclosure, processes, machines, manufacture, compositions of matter, means, methods, or steps, presently existing or later to be developed that perform substantially the same function or achieve substantially the same result as the corresponding embodiments described herein may be utilized.

**Claims**

1.   A method of dehydrogenating a hydrocarbon, the method comprising:

   initiating a dehydrogenation mode for a reactor to dehydrogenate the hydrocarbon;
   detecting a temperature in a catalyst bed of the reactor during the dehydrogenation mode;
   calculating mathematical time-derivatives of the detected temperature; and
   switching the reactor from the dehydrogenation mode to a regeneration mode to regenerate the catalyst of the catalyst bed, in response to an absolute value of the mathematical time-derivative of the detected temperature in the catalyst bed being less than a critical value.

2.   The method of claim 1, wherein the reactor is a fixed bed reactor.

3.   The method of any of claims 1 and 2, wherein the detecting comprises detecting a first temperature of a top portion of a catalyst bed and detecting a second temperature of a bottom portion of the catalyst bed of the reactor during the dehydrogenation mode.

4.   The method of claim 3, wherein the top portion includes a portion from a top of the catalyst bed to 15 % to 22% depth of the catalyst bed, and the bottom portion includes a bottom 10% to 20% depth of the catalyst bed.

5.   The method of claim 4, wherein the depth of the catalyst bed is about 190 cm, the top portion of the catalyst bed is a region from top of the catalyst bed to 30 cm in the catalyst bed below the top of the catalyst bed, and the bottom portion of the catalyst bed includes a region from 160 cm from top of the catalyst bed to the bottom of the catalyst bed.

6. The method of any of claims 3 to 5, wherein the calculating comprises calculating mathematical time-derivatives of the detected first temperature and mathematical time-derivatives of the detected second temperature.

7. The method of any of claims 3 to 6, wherein the switching comprises switching the reactor from the dehydrogenation mode to a regeneration mode to regenerate the catalyst bed, in response to (a) an absolute value of the mathematical time-derivative of the detected first temperature being less than a first critical value and/or (b) an absolute value of the mathematical time-derivative of the detected second temperature being less than a second critical value.

8. The method of any of claims 3 to 7, further comprising:
switching the reactor from the regeneration mode to the dehydrogenation mode in response to (c) an absolute value of the mathematical time-derivative of the detected first temperature being less than a third critical value and/or (d) an absolute value of the mathematical time-derivative of the detected second temperature being less than a fourth critical value.

9. The method of any of claims 1 to 8, wherein each of the initiating, detecting, calculating, and switching steps is conducted continuously.

10. The method of any of claims 1 to 9, wherein the hydrocarbon includes isobutane, n-butane, propane, ethane, methane, or combinations thereof.

11. A method of operating a reaction unit comprising one or more fixed bed reactors for dehydrogenating a hydrocarbon, the method comprising:

providing a programmable logic controller (PLC) comprising:

(i) a memory;
(ii) a database stored in the memory, the database comprising a first critical value, a second critical value, a third critical value and a fourth critical value;
(iii) one or more processors communicatively coupled to the memory, the one or more processors configured to switch each of the one or more fixed bed reactors between a dehydrogenation mode and a regeneration mode based on the first critical value, the second critical value, the third critical value, and the fourth critical value;

initiating, by the PLC, the dehydrogenation mode for one or more of the fixed bed reactors to dehydrogenate the hydrocarbon to produce a dehydrogenated hydrocarbon;
continuously detecting a first temperature of a top portion a catalyst bed of each of one or more of the fixed bed reactors;
continuously detecting a second temperature of a bottom portion of the catalyst bed of each of one or more of the fixed bed reactors during the dehydrogenation mode;
continuously calculating, by the PLC, mathematical time-derivatives of the detected first temperature and the detected second temperature for each of the fixed bed reactors;
switching, via the PLC, one or more of the fixed bed reactors from the dehydrogenation mode to the regeneration mode to regenerate the catalyst bed of each of the fixed bed reactors, in response to (a) an absolute value of the mathematical time-derivative of the detected first temperature being less than the first critical value and/or (b) an absolute value of the mathematical time-derivative of the detected second temperature being less than the second critical value; and
switching, via the PLC, one or more of the fixed bed reactors from the regeneration mode to the dehydrogenation mode, in response to (c) an absolute value of the mathematical time-derivative of the detected first temperature being less than the third critical value and/or (d) an absolute value of the mathematical time-derivative of the detected second temperature being less than the fourth critical value.

12. The method of claim 11, wherein the reaction unit is configured to dehydrogenate isobutane, wherein the reaction unit comprises 3-5 fixed bed reactors in parallel.

13. The method of claim 11, wherein the reaction unit is configured to dehydrogenate $C_3$ hydrocarbons, wherein the

reaction unit comprises 6-12 fixed bed reactors in parallel.

14. The method of any of claims 11 to 13, wherein the PLC is configured to control each of the one or more fixed bed reactors independently.

15. The method of any of claims 11 to 14, wherein the catalyst is selected from the group consisting of oxides or carbides of Al, Si, Ti, Zr, Zn, Ce, Sn, Mg, Ca, La, Cr, Cs, Ba, and combinations thereof.

**Patentansprüche**

1. Verfahren zum Dehydrieren eines Kohlenwasserstoffs, das Verfahren umfassend:

   Einleiten eines Dehydrierungsmodus für einen Reaktor, um den Kohlenwasserstoff zu dehydrieren;
   Erfassen einer Temperatur in einem Katalysatorbett des Reaktors während des Dehydrierungsmodus;
   Berechnen mathematischer Zeitableitungen der erfassten Temperatur; und
   Umschalten des Reaktors von dem Dehydrierungsmodus in einen Regenerationsmodus, um den Katalysator des Katalysatorbetts zu regenerieren, als Reaktion darauf, dass ein Absolutwert der mathematischen Zeitableitung der erfassten Temperatur in dem Katalysatorbett geringer als ein kritischer Wert ist.

2. Verfahren nach Anspruch 1, wobei der Reaktor ein Festbettreaktor ist.

3. Verfahren nach einem der Ansprüche 1 und 2, wobei das Erfassen das Erfassen einer ersten Temperatur eines oberen Abschnitts eines Katalysatorbetts und das Erfassen einer zweiten Temperatur eines unteren Abschnitts des Katalysatorbetts des Reaktors während des Dehydrierungsmodus umfasst.

4. Verfahren nach Anspruch 3, wobei der obere Abschnitt einen Abschnitt von einer Oberseite des Katalysatorbetts bis zu 15 % bis 22 % Tiefe des Katalysatorbetts umfasst und der untere Abschnitt eine Unterseite 10 % bis 20 % Tiefe des Katalysatorbetts umfasst.

5. Verfahren nach Anspruch 4, wobei die Tiefe des Katalysatorbetts etwa 190 cm beträgt, der obere Abschnitt des Katalysatorbetts ein Bereich von der Oberseite des Katalysatorbetts bis zu 30 cm in dem Katalysatorbett unterhalb der Oberseite des Katalysatorbetts ist und der untere Abschnitt des Katalysatorbetts einen Bereich von 160 cm von der Oberseite des Katalysatorbetts bis zu der Unterseite des Katalysatorbetts umfasst.

6. Verfahren nach einem der Ansprüche 3 bis 5, wobei das Berechnen das Berechnen mathematischer Zeitableitungen der erfassten ersten Temperatur und mathematischer Zeitableitungen der erfassten zweiten Temperatur umfasst.

7. Verfahren nach einem der Ansprüche 3 bis 6, wobei das Umschalten das Umschalten des Reaktors von dem Dehydrierungsmodus in einen Regenerationsmodus umfasst, um das Katalysatorbett zu regenerieren, als Reaktion darauf, dass (a) ein Absolutwert der mathematischen Zeitableitung der erfassten ersten Temperatur geringer als ein erster kritischer Wert ist und/oder (b) ein Absolutwert der mathematischen Zeitableitung der erfassten zweiten Temperatur geringer als ein zweiter kritischer Wert ist.

8. Verfahren nach einem der Ansprüche 3 bis 7, ferner umfassend:
   Umschalten des Reaktors von dem Regenerationsmodus in den Dehydrierungsmodus als Reaktion darauf, dass (c) ein Absolutwert der mathematischen Zeitableitung der erfassten ersten Temperatur geringer als ein dritter kritischer Wert ist und/oder (d) ein Absolutwert der mathematischen Zeitableitung der erfassten zweiten Temperatur geringer als ein vierter kritischer Wert ist.

9. Verfahren nach einem der Ansprüche 1 bis 8, wobei jeder der Schritte des Einleitens, Erfassens, Berechnens und Umschaltens kontinuierlich vorgenommen wird.

10. Verfahren nach einem der Ansprüche 1 bis 9, wobei der Kohlenwasserstoff Isobutan, n-Butan, Propan, Ethan, Methan oder Kombinationen davon einschließt.

11. Verfahren zum Betreiben einer Reaktionseinheit, umfassend einen oder mehrere Festbettreaktoren zum Dehydrieren eines Kohlenwasserstoffs, das Verfahren umfassend:

Bereitstellen einer speicherprogrammierbaren Steuerung (SPS), umfassend:

(i) einen Speicher;
(ii) eine Datenbank, die in dem Speicher gespeichert ist, die Datenbank umfassend einen ersten kritischen Wert, einen zweiten kritischen Wert, einen dritten kritischen Wert und einen vierten kritischen Wert;
(iii) einen oder mehrere Prozessoren, die mit dem Speicher kommunikativ gekoppelt sind, wobei der eine oder die mehreren Prozessoren konfiguriert sind, um jeden des einen oder der mehreren Festbettreaktoren basierend auf dem ersten kritischen Wert, dem zweiten kritischen Wert, dem dritten kritischen Wert und dem vierten kritischen Wert zwischen einem Dehydrierungsmodus und einem Regenerationsmodus umzuschalten;

Einleiten, durch die SPS, des Dehydrierungsmodus für einen oder mehrere der Festbettreaktoren, um den Kohlenwasserstoff zu dehydrieren, um einen dehydrierten Kohlenwasserstoff herzustellen;
kontinuierliches Erfassen einer ersten Temperatur eines oberen Abschnitts eines Katalysatorbetts von jedem von einem oder mehreren der Festbettreaktoren;
kontinuierliches Erfassen einer zweiten Temperatur eines unteren Abschnitts des Katalysatorbetts von jedem von einem oder mehreren der Festbettreaktoren während des Dehydrierungsmodus;
kontinuierliches Berechnen, durch die SPS, mathematischer Zeitableitungen der erfassten ersten Temperatur und der erfassten zweiten Temperatur für jeden der Festbettreaktoren;
Umschalten, über die SPS, eines oder mehrerer Festbettreaktoren von dem Dehydrierungsmodus in den Regenerationsmodus, um das Katalysatorbett jedes der Festbettreaktoren zu regenerieren, als Reaktion darauf, dass (a) ein Absolutwert der mathematischen Zeitableitung der erfassten ersten Temperatur geringer als der erste kritische Wert ist und/oder (b) ein Absolutwert der mathematischen Zeitableitung der erfassten zweiten Temperatur geringer als der zweite kritische Wert ist; und
Umschalten, über die SPS, eines oder mehrerer Festbettreaktoren von dem Regenerationsmodus in den Dehydrierungsmodus als Reaktion darauf, dass (c) ein Absolutwert der mathematischen Zeitableitung der erfassten ersten Temperatur geringer als der dritte kritische Wert ist und/oder (d) ein Absolutwert der mathematischen Zeitableitung der erfassten zweiten Temperatur geringer als der vierte kritische Wert ist.

12. Verfahren nach Anspruch 11, wobei die Reaktionseinheit konfiguriert ist, um Isobutan zu dehydrieren, wobei die Reaktionseinheit und 3-5 parallel geschaltete Festbettreaktoren umfasst.

13. Verfahren nach Anspruch 11, wobei die Reaktionseinheit konfiguriert ist, um $C_3$-Kohlenwasserstoffe zu dehydrieren, wobei die Reaktionseinheit 6-12 parallel geschaltete Festbettreaktoren umfasst.

14. Verfahren nach einem der Ansprüche 11 bis 13, wobei die SPS konfiguriert ist, um jeden von dem einen oder den mehreren Festbettreaktoren unabhängig zu steuern.

15. Verfahren nach einem der Ansprüche 11 bis 14, wobei der Katalysator aus der Gruppe ausgewählt ist, bestehend aus Oxiden oder Carbiden von Al, Si, Ti, Zr, Zn, Ce, Sn, Mg, Ca, La, Cr, Cs, Ba und Kombinationen davon.

## Revendications

1. Procédé de déshydrogénation d'un hydrocarbure, le procédé comprenant :

l'initiation d'un mode de déshydrogénation pour un réacteur destiné à déshydrogéner l'hydrocarbure ;
la détection d'une température dans un lit de catalyseur du réacteur pendant le mode de déshydrogénation ;
le calcul de dérivées temporelles mathématiques de la température détectée ; et
la commutation du réacteur du mode de déshydrogénation à un mode de régénération pour régénérer le catalyseur du lit de catalyseur, en réponse à une valeur absolue de la dérivée temporelle mathématique de la température détectée dans le lit de catalyseur étant inférieure à une valeur critique.

2. Procédé selon la revendication 1, dans lequel le réacteur est un réacteur à lit fixe.

3. Procédé selon l'une quelconque des revendications 1 et 2, dans lequel la détection comprend la détection d'une première température d'une partie supérieure d'un lit de catalyseur et la détection d'une seconde température d'une partie inférieure du lit de catalyseur du réacteur pendant le mode de déshydrogénation.

**4.** Procédé selon la revendication 3, dans lequel la partie supérieure comporte une partie allant d'un sommet du lit de catalyseur jusqu'à 15 % à 22 % de profondeur du lit de catalyseur, et la partie inférieure comporte 10 % à 20 % inférieurs de profondeur du lit de catalyseur.

**5.** Procédé selon la revendication 4, dans lequel la profondeur du lit de catalyseur est d'environ 190 cm, la partie supérieure du lit de catalyseur est une région allant du sommet du lit de catalyseur jusqu'à 30 cm dans le lit de catalyseur en dessous du sommet du lit de catalyseur, et la partie inférieure du lit de catalyseur comporte une région allant de 160 cm du sommet du lit de catalyseur jusqu'en bas du lit de catalyseur.

**6.** Procédé selon l'une quelconque des revendications 3 à 5, dans lequel le calcul comprend le calcul de dérivées temporelles mathématiques de la première température détectée et de dérivées temporelles mathématiques de la seconde température détectée.

**7.** Procédé selon l'une quelconque des revendications 3 à 6, dans lequel la commutation comprend la commutation du réacteur du mode de déshydrogénation à un mode de régénération pour régénérer le lit de catalyseur, en réponse à (a) une valeur absolue de la dérivée temporelle mathématique de la première température détectée étant inférieure à une première valeur critique et/ou (b) une valeur absolue de la dérivée temporelle mathématique de la seconde température détectée étant inférieure à une deuxième valeur critique.

**8.** Procédé selon l'une quelconque des revendications 3 à 7, comprenant en outre :
la commutation du réacteur du mode de régénération au mode de déshydrogénation en réponse à (c) une valeur absolue de la dérivée temporelle mathématique de la première température détectée étant inférieure à une troisième valeur critique et/ou (d) une valeur absolue de la dérivée temporelle mathématique de la seconde température détectée étant inférieure à une quatrième valeur critique.

**9.** Procédé selon l'une quelconque des revendications 1 à 8, dans lequel chacune des étapes d'initiation, de détection, de calcul et de commutation est effectuée de façon continue.

**10.** Procédé selon l'une quelconque des revendications 1 à 9, dans lequel l'hydrocarbure comporte isobutane, n-butane, propane, éthane, méthane, ou des combinaisons de ceux-ci.

**11.** Procédé de fonctionnement d'une unité de réaction comprenant un ou plusieurs réacteurs à lit fixe pour la déshydrogénation d'un hydrocarbure, le procédé comprenant :

la fourniture d'un automate programmable (PLC) comprenant :

(i) une mémoire ;
(ii) une base de données stockée dans la mémoire, la base de données comprenant une première valeur critique, une deuxième valeur critique, une troisième valeur critique et une quatrième valeur critique ;
(iii) un ou plusieurs processeurs couplés par communications avec la mémoire, le ou les processeurs étant configurés pour commuter chacun parmi le ou les réacteurs à lit fixe entre un mode de déshydrogénation et un mode de régénération en fonction de la première valeur critique, de la deuxième valeur critique, de la troisième valeur critique et de la quatrième valeur critique ;

l'initiation, par le PLC, du mode de déshydrogénation pour un ou plusieurs des réacteurs à lit fixe pour déshydrogéner l'hydrocarbure pour produire un hydrocarbure déshydrogéné ;
la détection continue d'une première température d'une partie supérieure d'un lit de catalyseur de chacun parmi un ou plusieurs des réacteurs à lit fixe ;
la détection continue d'une seconde température d'une partie inférieure du lit de catalyseur de chacun parmi un ou plusieurs des réacteurs à lit fixe pendant le mode de déshydrogénation ;
le calcul continu, par le PLC, de dérivées temporelles mathématiques de la première température détectée et de la seconde température détectée pour chacun des réacteurs à lit fixe ;
la commutation, par l'intermédiaire du PLC, d'un ou plusieurs des réacteurs à lit fixe du mode de déshydrogénation au mode de régénération pour régénérer le lit de catalyseur de chacun des réacteurs à lit fixe, en réponse à (a) une valeur absolue de la dérivée temporelle mathématique de la première température détectée étant inférieure à la première valeur critique et/ou (b) une valeur absolue de la dérivée temporelle mathématique de la seconde température détectée étant inférieure à la deuxième valeur critique ; et
la commutation, par l'intermédiaire du PLC, d'un ou plusieurs des réacteurs à lit du mode de régénération au mode de déshydrogénation, en réponse à (c) une valeur absolue de la dérivée temporelle mathématique de la première température détectée étant inférieure à la troisième valeur critique et/ou (d) une valeur absolue de la dérivée temporelle mathématique de la seconde température détectée étant inférieure à la quatrième valeur critique.

12. Procédé selon la revendication 11, dans lequel l'unité de réaction est configurée pour déshydrogéner de l'isobutane, dans lequel l'unité de réaction comprend 3 à 5 réacteurs à lit fixe en parallèle.

13. Procédé selon la revendication 11, dans lequel l'unité de réaction est configurée pour déshydrogéner des hydro-carbures en $C_3$, dans lequel l'unité de réaction comprend 6 à 12 réacteurs à lit fixe en parallèle.

14. Procédé selon l'une quelconque des revendications 11 à 13, dans lequel le PLC est configuré pour commander chacun parmi le ou les réacteurs à lit fixe indépendamment.

15. Procédé selon l'une quelconque des revendications 11 à 14, dans lequel le catalyseur est choisi dans le groupe constitué par des oxydes ou carbures d'Al, Si, Ti, Zr, Zn, Ce, Sn, Mg, Ca, La, Cr, Cs, Ba, et combinaisons de ceux-ci.

*FIG. 1*

200 —↘

```
┌─────────────────────────────────────────────────────────────┐
│   PROVIDE A PROGRAMMABLE LOGIC CONTROLLER (PLC) TO A          │── 201
│   HYDROCARBON DEHYDROGENATION SYSTEM                          │
└─────────────────────────────────────────────────────────────┘
                            │
                            ▼
┌─────────────────────────────────────────────────────────────┐
│   INITIATE A DEHYDROGENATION MODE FOR ONE OR MORE REACTORS OF │
│   THE DEHYDROGENATION SYSTEM TO DEHYDROGENATE A HYDROCARBON   │── 202
│   AND PRODUCE A DEHYDROGENATED HYDROCARBON HYDROCARBON        │
└─────────────────────────────────────────────────────────────┘
                            │
                            ▼
┌─────────────────────────────────────────────────────────────┐
│   DETECT, BY TEMPERATURE SENSORS, A FIRST TEMPERATURE OF A    │── 203
│   TOP PORTION OF A CATALYST BED FOR EACH REACTOR              │
└─────────────────────────────────────────────────────────────┘
                            │
                            ▼
┌─────────────────────────────────────────────────────────────┐
│   DETECT A SECOND TEMPERATURE OF A BOTTOM PORTION OF THE      │── 204
│   CATALYST BED FOR EACH REACTOR                               │
└─────────────────────────────────────────────────────────────┘
                            │
                            ▼
┌─────────────────────────────────────────────────────────────┐
│   CALCULATE, BY PLC 120, MATHEMATICAL TIME-DERIVATIVES OF THE │
│   DETECTED FIRST TEMPERATURE AND THE DETECTED SECOND          │── 205
│   TEMPERATURE FOR EACH REACTOR                                │
└─────────────────────────────────────────────────────────────┘
                            │
                            ▼
┌─────────────────────────────────────────────────────────────┐
│   SWITCH, VIA THE PLC, ONE OR MORE OF THE REACTORS FROM THE   │
│   DEHYDROGENATION MODE TO A REGENERATION MODE TO REGENERATE   │
│   THE CATALYST OF EACH OF THE REACTORS, IN RESPONSE TO (A) AN │
│   ABSOLUTE VALUE OF THE MATHEMATICAL TIME-DERIVATIVE OF THE   │
│   DETECTED FIRST TEMPERATURE BEING LESS THAN A FIRST CRITICAL │── 206
│   VALUE AND/OR (B) AN ABSOLUTE VALUE OF THE MATHEMATICAL      │
│   TIME-DERIVATIVE OF THE DETECTED SECOND TEMPERATURE BEING    │
│   LESS THAN A SECOND CRITICAL VALUE                           │
└─────────────────────────────────────────────────────────────┘
                            │
                            ▼
┌─────────────────────────────────────────────────────────────┐
│   SWITCH, VIA THE PLC, ONE OR MORE OF THE REACTORS FROM THE   │
│   REGENERATION MODE TO THE DEHYDROGENATION MODE, IN RESPONSE  │
│   TO (C) AN ABSOLUTE VALUE OF THE MATHEMATICAL TIME-DERIVATIVE│
│   OF THE DETECTED FIRST TEMPERATURE BEING LESS THAN A THIRD   │── 207
│   CRITICAL VALUE AND/OR (D) AN ABSOLUTE VALUE OF THE          │
│   MATHEMATICAL TIME-DERIVATIVE OF THE DETECTED SECOND         │
│   TEMPERATURE BEING LESS THAN A FOURTH CRITICAL VALUE         │
└─────────────────────────────────────────────────────────────┘
```

*FIG. 2*

FIG. 3A

FIG. 3B

FIG. 4A

FIG. 4B

FIG. 5A

EP 4 427 112 B1

*FIG. 5B*

**EP 4 427 112 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2298399 A1

- WO 2020183359 A1